# EUROPEAN PATENT APPLICATION

(11) **EP 1 779 790 A2**
(43) Date of publication of application: **02.05.2007**
(21) Application number: 06255516.4
(22) Date of filing: 26.10.2006
(51) Int. Cl.: A61B 17/15, A61B 19/00, A61B 17/17, A61F 2/46

(54) **Support for locating instrument guides**

(30) Priority: 27.10.2005 US 260454
(71) Applicant: DePuy Products, Inc., Warsaw, IN 46581 (US)
(72) Inventor: Wyss, Joseph G., Ft. Wayne, IN 46814 (US); Holm, Mara C., Lake Villa, IL 60046 (US)
(74) Representative: Belcher, Simon James

(57) **Abstract**

A surgical instrument system includes a cutting instrument, an anchoring structure, a guide structure and an articulating linkage. The anchoring structure can be fixed to a reference or benchmark, such as a location on the patient's bone. The articulating linkage connects the anchoring structure to the guide structure and includes a plurality of lockage ball joints arranged in series. Each ball joint provides for freedom of movement about three axes of rotation. The articulating linkage may include other structures allowing for additional rotational and translational degrees of freedom of movement for the guide structure with respect to the reference or benchmark. The system is useful in positioning, aligning and orienting a cutting block in joint arthroplasty, and may be useful in conjunction with computer assisted surgery. The system may also include computer navigation trackers for use in such computer assisted surgery.

## Description

The present invention relates to surgical instruments used to prepare a bone to receive a prosthetic implant, and more particularly, to such an instrument system used in conjunction with computer assisted surgery.

When a skeletal joint is damaged, whether as a result of an accident or illness, a prosthetic replacement of the damaged joint may be necessary to relieve pain and to restore normal use to the joint. Typically the entire joint is replaced by means of a surgical procedure that involves removal of the ends of the corresponding damaged bones and replacement of these ends with prosthetic implants. This replacement of a native joint with a prosthetic joint is referred to as a primary total-joint arthroplasty.

The surgical preparation of the bones during primary total-joint arthroplasty is a complex procedure. A number of bone cuts are made to effect the appropriate placement and orientation of the prosthetic components on the bones. In total knee arthroplasty, the joint gaps in extension and flexion must also be appropriate.

In the case of total knee arthroplasty, cutting guide blocks are used in creating the bone cuts on the proximal tibia and distal femur. The position, alignment and orientation of the cutting blocks are important in ensuring that the bone cuts will result in optimal performance of the prosthetic implant components. Generally, a tibial cutting block is positioned, aligned and oriented so that the cutting guide surface is in the optimal proximal-distal position, posterior slope, and varus-valgus orientation. Depending on the type of prosthetic implant system to be used, one or more cutting blocks are also positioned, aligned and oriented on the distal femur to ensure appropriate positioning of the distal femoral implant component and appropriate joint gaps.

A variety of alignment guides and cutting blocks have been provided in the prior art for use in preparing bone surfaces in primary total-knee arthroplasty, including alignment guides and cutting blocks used in preparing the proximal tibia and distal femur.

Prior art instrument sets with alignment guides include the Specialist® 2 instruments (DePuy Orthopaedics, Inc., Warsaw, IN) for use with DePuy Orthopaedics' P.F.C.® Sigma Knee System. The extramedullary tibial alignment guide for this instrument system includes an ankle clamp, a pair of telescoping alignment rods and a cutting block. The ankle clamp is affixed about the patient's ankle, without extending through the patient's soft tissue. Parts of this system are manually adjustable: the proximal-distal position of the cutting block is adjusted by sliding the telescoping rods and then locking the rods in the desired position; posterior slope is set at the ankle by sliding the distal end of the alignment rod in an anterior-posterior direction to thereby pivot the cutting block into the desired orientation; varus-valgus slope is set by pivoting the cutting block so that the alignment guide pivots about a rod at the ankle clamp.

US-6,490,114 discloses a tibial plateau resection guide. This system also uses an ankle clamp and extension rods to set the position and orientation of the cutting block. US-5,451,228 also utilizes an ankle clamp but allows for angular orientation in the anterior-posterior plane to predetermined angular orientations using a thumb actuated slide mechanism; the device is however limited to predetermined angular settings. US-6,685,711 and US-6,595,997 disclose an apparatus and method for resecting bone that provides for aligning a resection guide in three degrees of freedom.

The present invention provides a surgical instrument system that can be used to efficiently and accurately set the position, alignment and orientation of cutting blocks and other surgical instruments.

In one aspect, the present invention meets these objectives by providing a surgical instrument system for resecting a bone during joint arthroplasty. The system comprises a cutting instrument, an anchoring structure, a guide structure and an articulating linkage. The guide structure guides the path of the cutting instrument. The articulating linkage connects the anchoring structure to the guide structure, and includes a plurality of lockable ball joints arranged in series. Each ball joint provides for freedom of movement about three axes of rotation.

In another aspect, there is provided surgical instrument system for resecting a bone during joint arthroplasty, the system comprising: a cutting instrument; an anchoring structure; a guide structure for guiding the path of the cutting instrument; and an articulating linkage attachable to the anchoring structure to the guide structure. The articulating linkage includes a plurality of lockable ball joints arranged in series, each ball joint providing for freedom of movement about three axes of rotation.

The system may further comprise a second guide structure for guiding the path of the cutting instrument, the articulating linkage being attachable to the second guide structure.

The first and second guide structures may be selected from the group consisting of a proximal tibial cutting block, a distal femoral cutting block, a distal tibial cutting block, a proximal femoral cutting block and a guide track.

The system may further comprise a plurality of computer navigation trackers.

The guide structure may have two translational degrees of freedom of movement and at least eight rotational degrees of freedom of movement with respect to the anchoring structure.

Embodiments of the invention will now be described by way of example with reference to the accompanying drawings, wherein like numbers denote like parts throughout and in which:
FIG. 1 is a perspective view of a first embodiment of a surgical instrument system embodying the principles of the present invention, shown mounted to a patient's femur;
FIG. 1 A is a plan view of an example of a surgical instrument that may be used with the system illustrated in FIG. 1;
FIG. 2 is an end view of an embodiment of an anchoring structure that may be used in the present invention, the anchoring structure being shown mounted to a patient's femur;
FIG. 3 is an elevation, from the lateral side of the bone, of the anchoring structure of FIG. 2;
FIG. 4 is a top plan view, from the anterior side of the bone, of the anchoring structure of FIGS. 2-3;
FIG. 4A is an elevation of the pin-clamping half of one of the anchoring clamp assemblies of the anchoring structure;
FIG. 4B is an elevation of the bar-clamping half of one of the anchoring clamp assemblies of the anchoring structure;
FIG. 5 is an elevation of the movable clamp assembly and bi-axial articulating assembly of the system of FIG. 1, taken from a proximal or distal side;
FIG. 6 is an exploded elevational view of the movable clamp assembly and bi-axial articulating assembly of FIG. 5;
FIG. 7 is an exploded perspective view of the movable clamp assembly and bi-axial articulating assembly of FIGS. 5-6;
FIG. 8 is an elevation of the movable clamp assembly and bi-axial articulating assembly of FIGS. 5-7, taken from a medial or lateral side;
FIG. 9 is a top plan view of the movable clamp assembly and bi-axial articulating assembly of FIGS. 5-8;
FIG. 9A is an exploded elevational view of an alternative embodiment of a movable clamp assembly and bi-axial articulating assembly that may be used in the instrument system of the present invention;
FIG. 9B is an exploded perspective view of the movable clamp assembly and bi-axial articulating assembly of FIG. 9A;
FIG. 10 is a top plan view of a housing and articulating ball joints of the articulating linkage of the system of FIG. 1;
FIG. 11 is a view similar to FIG. 10, with the housing shown in cross-section and with the ball joints shown in one unlocked position;
FIG. 12 is a cross-section taken along line 12-12 of FIG. 10;
FIG. 13 is a view similar to FIG. 11, shown with the ball joints in a second unlocked position;
FIG. 14 is a view similar to FIGS. 11 and 13, shown with the ball joints locked in a selected position;
FIG. 15 is an end view of the housing and first ball joint in the locked position of the FIG. 14;
FIG. 16 is an elevation of the guide structure of the embodiment of FIG. 1 shown mounted on a first fully articulatable arm;
FIG. 17 is an end view of the guide structure and first fully articulatable arm of FIG. 16, taken along line 17-17 of FIG. 16;
FIG. 18 is a cross-section of the guide structure and first fully articulatable arm of FIGS. 16-17, taken along line 18-18 of FIG. 16, shown with cam in a locked position to fix the position of the guide structure on the first fully articulatable arm;
FIG. 19 is a cross-section similar to FIG. 18, shown with the cam in a locked position to allow translational movement of the guide structure on the first fully articulatable arm;
FIG. 20 is an end view of the first fully articulatable arm;
FIG. 21 is an exploded view of the guide structure, cam and first fully articulatable arm of FIGS. 16-19;
FIG.22 is an exploded perspective view of the guide structure of FIGS. 16-19 and 21;
FIG. 23 is an elevation of alternative embodiment of an intermediate member for use as part of an articulating linkage, shown with the fully articulatable rod in an extended position;
FIG. 24 is a view of the alternative intermediate member of FIG. 23, shown with the fully articulatable rod in a retracted position;
FIG. 25 is a perspective view of the alternative intermediate member of FIGS. 23-24;
FIG. 26 is an end view of the alternative intermediate member of FIGS. 23-25;
FIG. 27 is a view of the alternative intermediate member of FIGS. 23-26 with the body of the alternative intermediate member shown in longitudinal cross-section;
FIG. 28 is a transverse cross-section of the alternative intermediate member of FIGS. 23-26, shown with the handle in an unlocked position;
FIG. 29 is a transverse cross-section similar to FIG. 28, shown with the handle in a locked position;
FIG. 30 is an elevation of a portion of one of the curved interior side walls of the alternative intermediate member of FIGS. 23-28;
FIG. 31 is a top plan view of a portion of the fully articulatable arm used in combination with the alternative intermediate member of FIGS. 23-28;
FIG. 32 is a side elevation of the fully articulatable arm of FIG. 31;
FIG. 33A is a top plan view of an alternative embodiment of a housing and locking mechanism for the ball joints of the articulating linkage;
FIG. 33B is a cross-section through the housing of the embodiment of FIG. 33A, with the ball joints shown in an unlocked position;
FIG. 34 is an anterior view of a femur and an alternative embodiment of the instrument system of the present invention, shown with a jointed intermediate link in the articulating linkage, and shown with a guide track used as a guide structure to guide the path of an alternative cutting instrument;
FIG. 35 is a schematic view of an embodiment of the instrument system of the present invention used to position and orient a distal femoral cutting block;
FIG. 36 is a schematic view of an embodiment of the instrument system of the present invention in combination with computer navigation trackers used to position and orient a proximal tibial cutting block;
FIG. 37 is a schematic view of the instrument system of FIG. 36, shown with a distal femoral cutting block attached to the articulating linkage, illustrating that the instrument system can be used to position and orient a number of cutting blocks during a single procedure without changing the position of the anchoring structure;
FIG. 38 is a schematic anterior view of the distal tibia, shown with an embodiment of the instrument system of the present invention used to position and orient a cutting block for use in ankle arthroplasty; and
FIG. 39 is a schematic view anterior view of a proximal femur, shown with an embodiment of the instrument system of the present invention used to position and orient a cutting block for use in hip arthroplasty.

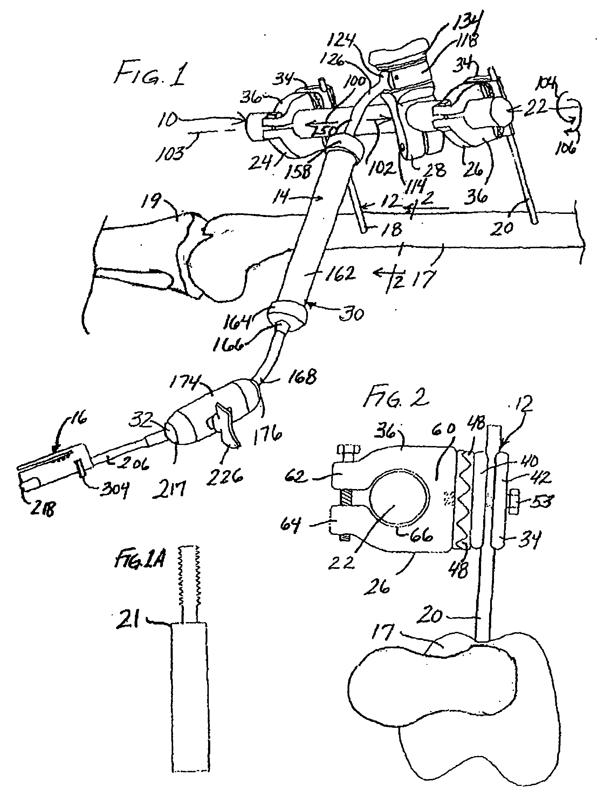

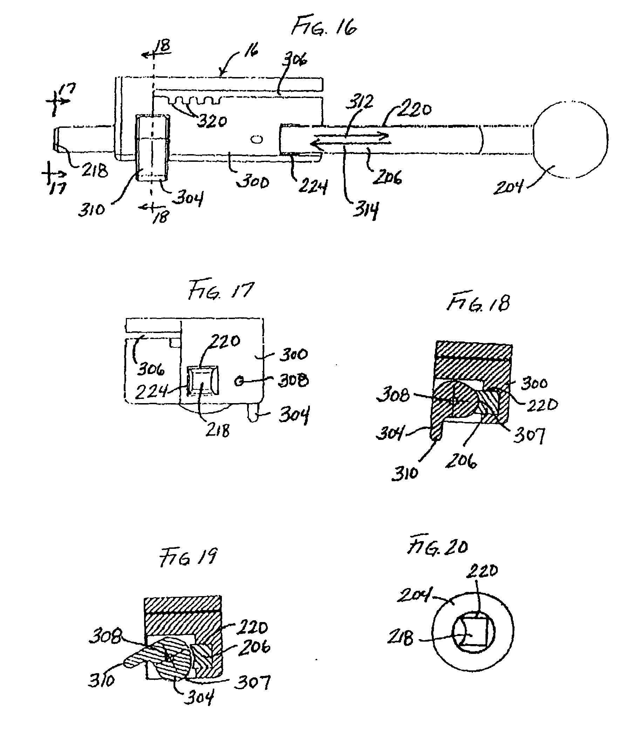

A first embodiment of an instrument system illustrating the principles of the present invention is illustrated at 10 in FIGS. 1-22. The first illustrated instrument system 10 comprises three main parts: an anchoring structure 12 that is mounted to a reference such as a bone shown at 17 in FIG. 1; an articulating linkage 14 that is connected to the anchoring structure 12; and a guide structure 16 that is connected to the articulating linkage. With this instrument set, the surgeon can rigidly fix the anchoring structure 12 to one of the patient's bones, move the guide structure 16 into a desired position and then lock the articulating linkage 14 to hold the guide structure 16 in the desired position. The guide structure 16 may then be fixed to the bone 17 or to a neighboring bone such as tibia 19 with fixation pins or the like if desired. The guide structure 16 may then be used to guide the path of a surgical instrument, such as a reciprocating saw shown at 21 in FIG. 1A, a milling device, a burr or a drill, for example.

As discussed in more detail below, the articulating linkage 14 includes a plurality of lockable ball joints arranged in series. Each ball joint provides for freedom of movement about three axes of rotation. Once the desired orientation of the guide structure is achieved, each ball joint can be locked to set the position, alignment and orientation of the guide structure. The articulating linkage provides for substantial freedom of movement of the guide structure 16 for efficient and accurate placement of the guide structure intraoperatively.

As discussed in more detail below, each structure 12, 14, 16 of the instrument set may comprise assemblies of elements. For example, in the first illustrated embodiment, the anchoring structure 12 comprises an assembly of a plurality of pins 18, 20, an anchoring bar 22, and a pair of anchoring clamp assemblies 24, 26 for fixing the anchoring bar 22 to the pins 18, 20 (see FIGS. 1-4). The articulating linkage 14 of the first illustrated instrument system 10 includes a movable clamp assembly 28, a bi-axial assembly 30 and a multi-axial assembly 32. The multi-axial assembly 32 comprises a plurality of lockable ball joints 176, 217 arranged in series. Each ball joint provides for freedom of movement about at least three axes of rotation. All of the movable elements 28, 30, 32 of the first illustrated instrument system 10 can be locked to thereby fix the position, alignment and orientation of the guide structure 16. The guide structure 16 can itself comprise an assembly as well. Each of the illustrated assemblies of elements is described in more detail below.

It should be understood that the anchoring structure 12, articulating linkage 14 and guide structure 16 may comprise fewer or more elements than those described below. In addition, as described in more detail below, some of these structures could be constructed as unitary components, rather than as assemblies of components.

First considering the elements of the anchoring structure 12 of the first illustrated instrument system 10, the pins 18, 20 may comprise standard surgical pins or wires used in orthopaedic surgery. The pins 18, 20 may be made of any standard surgical grade material such as stainless steel, and should have sufficient size and strength to support the weight of the articulating linkage 14 of the instrument system as well as the guide structure 16 of the instrument set. For example, it is anticipated that stainless steel pins having a diameter of 5 mm. and an overall length of 20 cm. and with pointed ends should be usable with the present invention. It should be understood that these materials and dimensions are provided as examples only; the present invention is not limited to any particular material or dimension unless expressly set forth in the claims.

The anchoring bar 22 of the anchoring structure 12 of the first illustrated instrument system 10 comprises a rod of any suitable surgical grade material, such as stainless steel. The bar 22 may be made of a material that can be sterilized by commercially available sterilization techniques without losing its strength. It may be desirable to make the anchoring bar out of a material that is radiolucent or radiotransparent so that radiographs may be taken intraoperatively without interference from the components of the anchoring structure 12. To decrease the overall weight of the system, it may be desirable to make the anchoring bar 22 out of a hollow tubular material such as stainless steel or out of a lightweight plastic material. For use in knee arthroplasty, the anchoring bar 22 may have a length of about 30 cm. and a diameter of about 18 mm., for example. The illustrated anchoring bar 22 is cylindrical in shape. It should be understood that these materials, dimensions and shape are provided as examples only; the present invention is not limited to any particular material, shape or dimension unless expressly set forth in the claims.

The anchoring bar 22 of the anchoring structure 12 of the first illustrated instrument system 10 is connected to the two pins 18, 20 through the two anchoring clamp assemblies 24, 26. Each of the illustrated anchoring clamp assemblies 24, 26 are the same; one of these clamp assemblies 24 is described below, and it should be understood that the description applies to both illustrated anchoring clamp assemblies 24, 26.

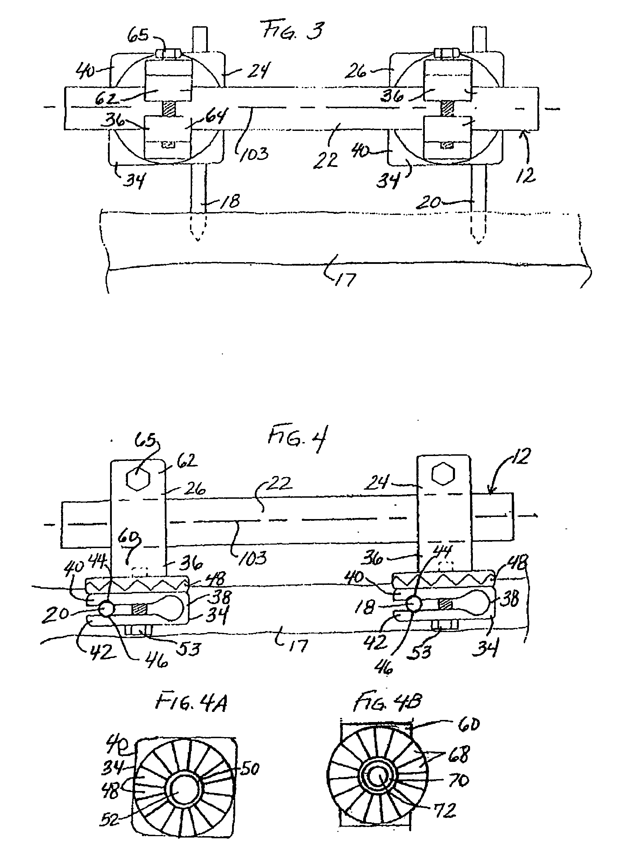

As shown in FIGS. 1-4, anchoring clamp assembly 24 comprises a pin-clamping half 34 and a bar-clamping half 36. As shown in FIG. 4, the pin-clamping half 34 of the clamp assembly 24 includes a bridge section 38 with integral spaced legs 40, 42. The bridge section 38 has a reduced thickness in the illustrated embodiment, allowing the spaced legs 40, 42 to flex for clamping the pins or wires. The spaced legs 40, 42 include parallel semicylindrical grooves 44, 46 sized and shaped to receive a portion of one of the pins 18, 20 longitudinally.

As shown in FIGS. 4 and 4A, one of the spaced legs 40 in the illustrated pin-clamping half 34 of the anchoring clamp assembly 24 includes a plurality of teeth 48 extending radially outward from a stub 50 with a fastening hole 52 for fastening the pin-clamping half to the bar-clamping half 36. The pin-clamping half 34 of the anchoring clamp assembly 24 may be fastened to the bar-clamping half 36 with a bolt, such as that shown at 53 in FIGS. 2-4. As shown in FIG. 2, the bar-clamping half 36 of the illustrated anchoring clamp assembly 24 includes a body 60 with a pair of spaced legs 62, 64. The body 60 has a substantially cylindrical groove 66 sized and shaped to receive a segment of the anchoring bar 22. The spaced legs 62, 64 include co-axial bores to receive a bolt 65 to tighten and loosen the legs 62, 64 about the anchoring bar 22.

As shown in FIGS. 4 and 4B, one surface of the illustrated body 60 has a fixed circular portion 66. The fixed circular portion 66 includes a plurality of teeth 68 extending radially outward from a stub 70 with a fastening hole 72 for fastening the bar-clamping half 36 to the pin-clamping half 34. To fasten the two halves 34, 36 together, the stub of one of the halves (such as stub 70 of half 36) may be received in the hole of the other half (such as hole 52 in half 34); the teeth 48 and 68 will mesh together, and the two halves 34, 36 can be locked together with the bolt 53. The intermeshed teeth 48, 68 will prevent relative rotation between the two halves 34, 36.

Although the illustrated embodiments of anchoring structures 12 are for mounting directly to the patient's bone, it should be understood that the articulating linkage 14 and guide structure 16 described below may be used with other types of anchoring structures. For example, the articulating linkage 14 described below could be used with a commercially available ankle clamp, in which case a suitable connection between the ankle clamp and the articulating linkage 14 should be provided, allowing for relative movement between the articulating linkage 14 and the anchoring structure 12. Anchoring the articulating linkage 14 and guide structure 16 to the patient's limb (e.g. leg) is advantageous in that if the patient's limb moves during the procedure, the positions of the articulating linkage 14 and guide structure 16 relative to the limb remain fixed. However, in some applications, other devices or fixtures could be used as the anchoring structure. For example the anchoring structure could comprise a portion of an operating room table or other fixture in the operating room when the system 10 is used in conjunction with computer assisted surgery.

Next considering the elements of the articulating linkage 14 of the first illustrated instrument system 10, the movable clamp assembly 28 is mounted on the anchoring bar 22. As indicated by arrows 100, 102 in FIG. 1, the illustrated movable clamp assembly 28 can be moved linearly along the longitudinal axis 103 of the anchoring bar 22 to a desired translational position and then fixed in the desired translational position along the length of the anchoring bar 22. The illustrated movable clamp assembly 28 can also be rotated about the longitudinal axis 103 of the anchoring bar 22, as indicated by arrows 104, 106 to a desired rotational position along the anchoring bar 22 and then fixed in the desired rotational position. Generally, the illustrated movable clamp assembly 28 can be fixed in the desired translational and rotational positions simultaneously.

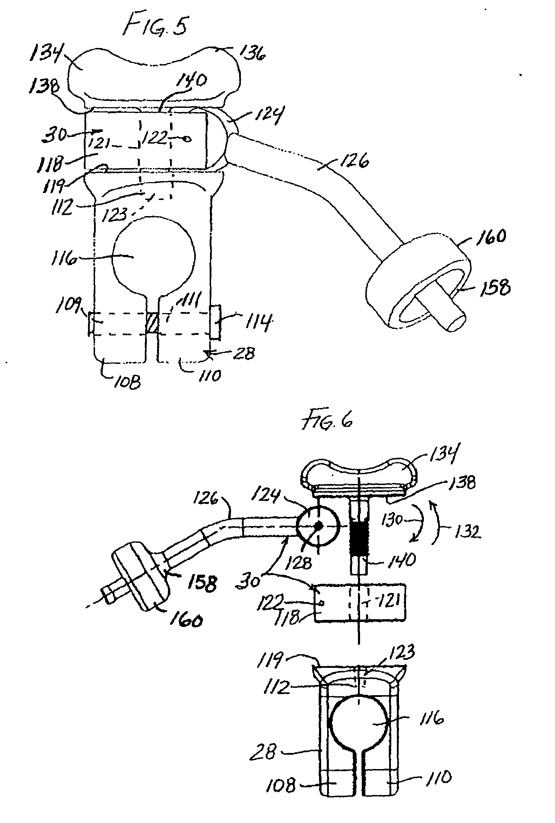

As shown in FIGS. 5-7, the illustrated movable clamp assembly 28 comprises a pair of spaced arms 108, 110 joined by a bridge section 112. The spaced arms 108, 110 substantially surround a portion of the anchoring bar 22. The spaced arms 108, 110 can be tightened around the bar 22 to fix the clamp assembly's linear and rotational positions by tightening bolt 114.

The spaced arms 108, 110 of the movable clamp assembly 28 and a portion of the bridge section 112 have a substantially cylindrical cut-out 116 having a diameter substantially the same as the diameter of the anchoring bar 22 for receiving the anchoring bar. The ends of the spaced arms 108, 110 have co-axial bores 109, 111 (shown in phantom in FIG. 5) to receive the bolt 114 to move the arms 108, 110 closer together to lock the arms on the anchoring bar 22.

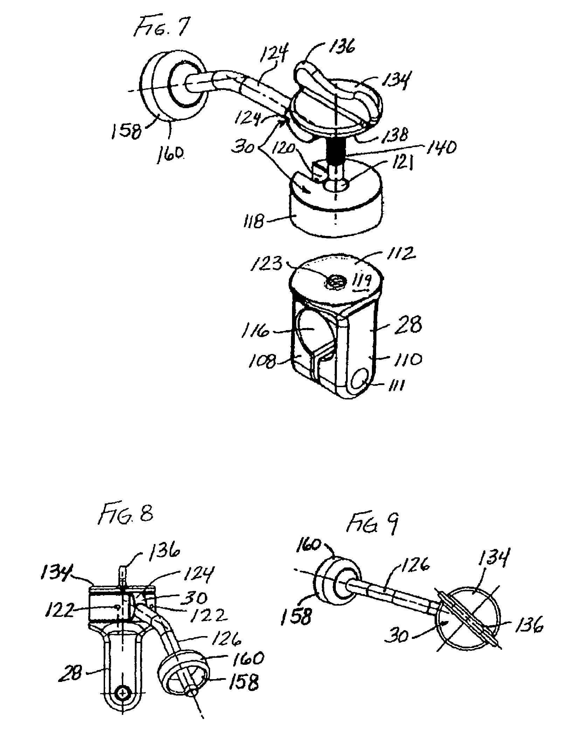

As shown in FIGS. 5-9, the movable clamp assembly 28 is mounted to the bi-axial articulating assembly 30. The bi-axial articulating assembly 30 includes a ring 118 positioned upon a flat circular surface 119 of the bridge section 112 of the movable clamp assembly 28. As shown in FIG. 7, the ring 118 includes a radial slot 120 and a central longitudinal bore 121. As shown in FIG. 6, the ring 118 also includes a transverse bore 122 intersecting the radial slot 120.

The radial slot 120 of the ring 118 receives a disc-shaped portion 124 of a pivotable arm 126. The pivotable arm 126 is mounted to the ring 118 through a transverse pin 127 extending through the transverse bore 122 of the ring 118 and through a bore 128 (see FIG. 6) in the center of the disc-shaped portion 124 of the pivotable arm 126. The pivotable arm 126 can be pivoted or rotated on the pin in the directions shown by arrows 130, 132 in FIG. 6 until a desired position is reached and then locked in the desired position.

To lock the pivotable arm 126 in the desired position, the bi-axial articulating assembly 30 includes a locking plate 134. The locking plate 134 includes a handle portion 136, a flat surface 138 and an extension 140. The extension 140 is partially threaded, and extends through co-axial central longitudinal bores 121, 123 in the ring 118 and bridge section 112. The bore 123 of the bridge section is also threaded. When assembled, the extension 140 extends through the central bore 121 of the ring 118 and is received in and threadedly engages the bore 123 in the bridge section 112. When the locking plate 134 is loose, the ring 118 is free to rotate about the central longitudinal axis of the extension 140. To lock the movable arm 126 in a desired position, the handle portion 136 of the locking plate 134 is turned so that the flat surface 138 of the locking plate 134 bears against the ring 118 and disc-shaped portion 124 of the pivotable arm 126 to lock them in position.

Thus, there are two rotational degrees of freedom of movement of the pivotable arm 126 with respect to the movable clamp 28: the pivotable arm 126 is rotatable about the transverse pin 127 and rotatable about the extension 140 to a desired position. Once the desired position is reached, the pivotable arm 126 can be locked in position with respect to the movable clamp assembly 28 by turning the locking plate 134. And since the movable clamp assembly 28 has two degrees of freedom of movement with respect to the stationary bar 22 (one rotational and one translational), the pivotable arm 126 has four degrees of freedom of movement with respect to the stationary bar 22 (and the bone 17 to which the stationary bar is fixed): three rotational and one translational.

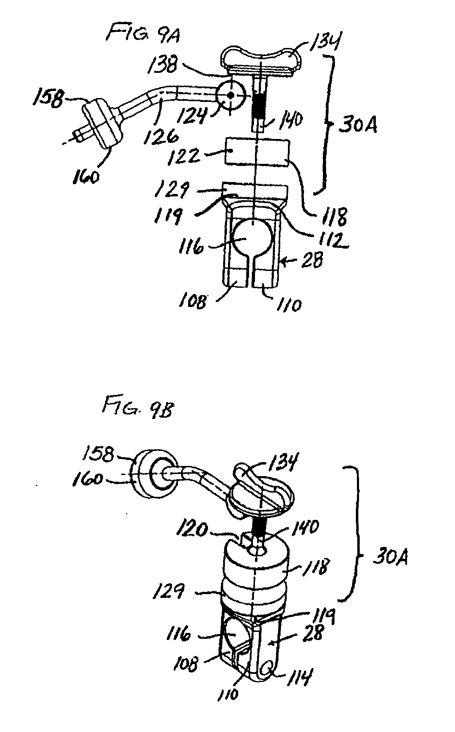

It should be understood that the design of a suitable bi-axial articulating subassembly and moveable clamp subassembly may vary from that illustrated in FIGS. 1 and 5-9. For example, an alternative bi-axial articulating subassembly 30A is shown in FIGS. 9A-9B at 30A. In this example, an additional disc 129 may be inserted between the ring 118 and the flat top surface 119 of the bridge section 112 of the movable clamp assembly 28. Otherwise, the elements of the embodiment of FIGS. 9A-9B are the same as those in FIGS. 5-9, as indicated by the use of common reference numbers.

The clamp assemblies 24, 26, movable clamp assembly 28 and bi-axial articulating assembly 30 may be made out of any standard surgical grade material. For example, a metal such as stainless steel could be used. If desired, some components of each assembly, or the entire assembly, could be made of other materials, such as a surgical grade plastic or composite material, such as carbon fiber material. They should preferably be made of a material that can be sterilized by conventional sterilization methods and that will retain their stiffness under the weight of the articulating linkage and guide structure during use. It may be desirable to make some or all of the components of the assemblies 24, 26, 28 out of a material that is radio-transparent or radio-lucent so that the assemblies do not block relevant portions of the patient's anatomy intra-operatively when radiographs are taken. To reduce the weight of the instrument set, some or all of the components of the assemblies 24, 26, 28 may be made of a lightweight material such as a carbon fiber-polymer composite.

As shown in FIGS. 5-9, the pivotable arm 126 of the bi-axial articulating assembly has a bar portion 150 that extends outward from the annular disc portion 124. In the illustrated embodiment, the bar portion 150 comprises two segments 152, 154 meeting at an obtuse angle. It should be understood that the shape of the bar portion 150 is provided by way of example only; the bar portion 150 could be curved or straight or could include addition linear segments meeting at a variety of angles.

As shown in FIGS. 5-6 and 8, the end of the bar portion 150 of the pivotable arm 126 opposite the annular disc portion 124 extends through a cylindrical sleeve portion 156 of a connector 158 to mount the pivotable arm 126 to the connector 158. The connector 158 includes an enlarged diameter portion 160 with a cylindrical flange. The cylindrical flange encircles one end of an intermediate link 162 to fix the connector 158 to the intermediate link 162. Thus, the connector 158 serves to connect one end of the pivotable arm 126 to one end of the intermediate link 162. This connection is a fixed one in the first illustrated embodiment; there is no relative linear or rotational movement between the pivotable arm 126 and the intermediate link 162 so that rotational and translational movement of the pivotable arm 126 results in rotation and translation of the intermediate link 162 as well.

Although the present invention is not limited to any particular size of intermediate link, for use in knee replacement surgery, it may be desirable to use an intermediate link 162 having a length of about 140 mm (5.5 inches) and an outer diameter of about 19 mm (0.75 inches). The intermediate link 162 of the first illustrated embodiment may comprise a solid bar or a hollow tube, for example. As discussed in more detail below, alternative designs for the intermediate link may be used.

The intermediate link 162 and connector 158 may be made of any commercially available surgical grade material, such as metal, plastic or composite material such as a carbon fiber material. They should preferably be made of a material that can be sterilized by conventional sterilization methods and that will retain their stiffness under the weight of the articulating linkage and guide structure during use. It may be desirable to make the intermediate link 162 and connector 158 out of a material that is radio-transparent or radio-lucent so that the intermediate link would not block relevant portions of the patient's anatomy intra-operatively when radiographs are taken. To reduce the weight of the instrument set, the intermediate link 162 and connector 158 may be made of a lightweight material such as a carbon fiber-polymer composite.

The opposite end of the intermediate link 162 is attached to a second connector 164, similar in structure and material to the first connector 158 described above. The second connector 164 includes a cylindrical sleeve portion 166 that receives a free end of a first fully articulatable arm 168 to mount the first fully articulatable arm 168 to the second connector 164. This mounting is a fixed one so that rotational or translational movement of the pivotable arm 126 results in the same rotational or translational movement of the first fully articulatable arm 168.

The illustrated first fully articulatable arm 168 of the first embodiment comprises a bar or tube portion 170 that has two segments meeting at an obtuse angle. It should be understood that the bar or tube portion may have other shapes as well; it may be straight, curved, or may comprise a combination of shapes.

Opposite the end received in the connector 164, the first fully articulatable arm 168 has a spherically-shaped portion 172. The spherically-shaped portion 172 of the arm 168 is received in a housing 174 to define a first articulating ball joint 176. The reference to arm 168 as being fully articulatable refers to the fact that the ball joint 176 allows for rotation of the arm 168 about at least three different axes.

The housing 174 in the illustrated embodiment includes a body 178 and a first end cap 180. The illustrated body 178 includes a generally cylindrical outer periphery 182 and an inner longitudinal bore 184. The bore 184 may be concentric with the outer periphery 182. The first end cap 180 may have any suitable shape capable of containing the spherically-shaped portion 172 of the first fully articulatable arm 168 while allowing a desired range of relative rotational motion between the housing 174 and the first articulatable arm 168.

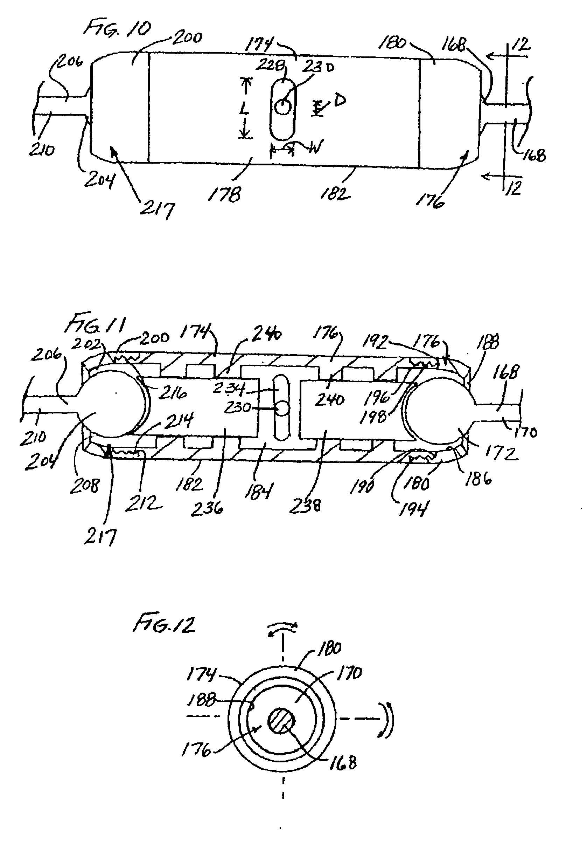

As shown in FIG. 11, the first end cap 180 includes a concave inner periphery 186 for cooperation with the spherically-shaped portion 172 of the first fully articulatable arm 168. The first end cap 180 further defines an opening 188 through which a part of the first fully articulatable arm 168 extends. The opening 188 in the illustrated embodiment is circular, with a diameter slightly less than the diameter of the spherically-shaped portion 172 of the first fully articulatable arm 168 so that the first fully articulatable arm 168 is held within the housing 174 while being allowed a substantial range of motion.

The illustrated first end cap 180 further includes a body opening 190 for receiving an end 192 of the body 178 of the housing 174. The first end cap 180 and body 178 may be secured together in any suitable way, for example, by a series of pins, a groove and lip, or, as shown in FIG. 11, by internal threads 194 formed on the first end cap 180 adjacent the body opening 190. The internal threads 194 of the first end cap 180 matingly engage external threads 196 formed on a first hub 198 of the body 178.

In the illustrated embodiment, the housing 174 also includes a second end cap 200. The second end cap 200 is similar to the first end cap 180 and includes a concave inner periphery 202 for cooperation with a spherically-shaped portion 204 of a second fully articulatable arm 206. The second cap 200 also has an opening 208 through which a rod or tube portion 210 of the second fully articulatable arm 206 extends. The inner periphery of the illustrated second cap 200 includes internal threads 212 that mate with external threads 214 formed on a second hub 216 of the body 178 of the housing 174. The spherically-shaped portion 204 of the second fully articulatable arm 206 and the housing 174 define a second multi-axial ball joint 217.

As illustrated in FIG. 1, the second fully articulatable arm 206 includes an end 218 opposite the spherically shaped end 204. As shown in FIG. 20, between the two ends 218, 204, the illustrated second fully articulatable arm 206 includes at least one flat surface 220 along a substantial part of its length; in the illustrated embodiment, the end segment 218 of the second fully articulatable arm has three flat surfaces. A portion of the second fully articulatable arm that has such flat surfaces 220 is received in a similarly-shaped bore 224 in the guide structure 16 (see FIGS. 16-19).

As shown in FIG. 1, the housing 174 also carries an actuator 226 for locking and unlocking the ball joints 176, 217. In the illustrated embodiment, a single actuator 226 is capable of simultaneously locking and unlocking the two ball joints 176, 217 through action upon a pair of movable pistons held within the body 178 of the housing 174.

In the first illustrated instrument, the body 178 of the housing 174 includes a transverse opening 228 through which a shaft 230 of the actuator 226 is rotatably fitted. Preferably, to accommodate component tolerances and the resultant tolerance stack of the components of the ball joints 176, 217, the transverse opening 228 may be sized to provide additional clearance between the transverse opening 228 and the shaft 230. The clearance accommodates the tolerances to that the shaft is not limited in its motion axially to the body.

For example, and as shown in FIG. 10, the body transverse opening 228 may be oval and defined by an opening length L that is substantially greater than the opening width W. The opening length L is made sufficiently larger than the diameter D of the shaft 230 so that the shaft does not impinge upon the body 178.

The actuator 226 in the first illustrated embodiment further includes a handle 232 and a force-transferring feature. The handle 232 is provided for turning the shaft 230 for locking and releasing the two ball joints 176, 217. The force-transferring feature is provided for translating the turning of the shaft 230 into a force acting to limit movement of the first and second fully articulatable arms 168, 206.

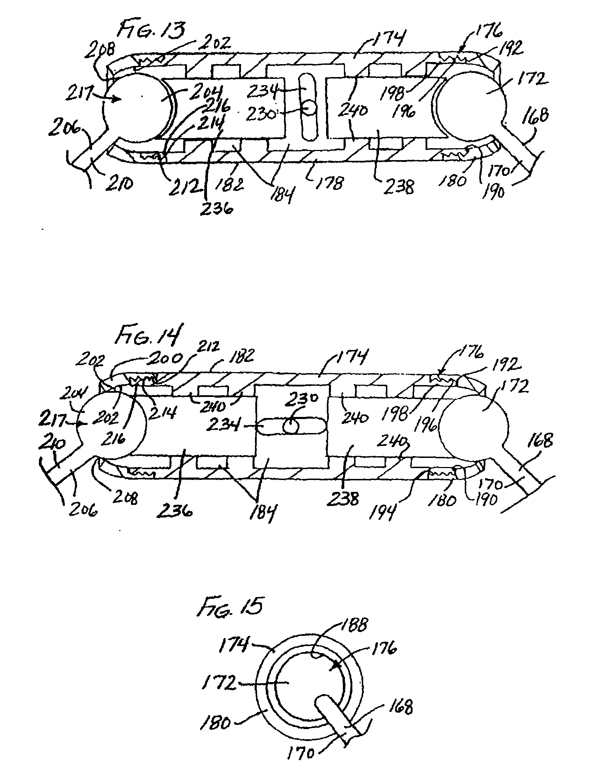

As shown in FIGS. 11 and 13-14, the force-transferring feature in the first illustrated embodiment comprises a cam 234 and a pair of pistons 236, 238 held within the housing 174. The cam 234 controls the movement and position of the two pistons 236, 238 within the housing 174. For example, as shown in FIGS. 11 and 13, when the cam 234 is in an unlocked position, the cam 234 does not contact the pistons 236, 238; the pistons 236, 238 are slightly spaced from the spherically-shaped end portions 172, 204 of the fully articulatable arms 168, 206, and the arms 168, 206 can be rotated about multiple axes to multiple rotational positions, as can be seen, for example, by comparing the positions of the tube portions 170, 210 of the fully articulatable arms 168, 206. As the shaft 230 is rotated about its central longitudinal axis, the cam 234 is turned from the position shown in FIGS. 11 and 13 to the position shown in FIG. 14. As the cam 234 turns, it advances the two pistons 236, 238 in opposite directions toward the spherically-shaped portions 172, 204. In particular, the first piston 236 advances from a first position (shown in FIGS. 11 and 13) to a second position (shown in FIG. 14). Similarly the second piston 238 advances from a first position (shown in FIGS. 11 and 13) to a second position (shown in FIG. 14).

When the first piston 236 and the second piston 238 are in their second positions shown in FIG. 14, an outer face of the first piston 236 engages the spherically-shaped portion 204 of the second fully articulatable arm 206 and an outer face of the second piston 238 simultaneously engages the spherically-shaped portion 172 of the first fully articulatable arm 168, thereby simultaneously locking the first and second fully articulatable arms 168, 206 in position. Thus, the first illustrated instrument provides for simultaneous locking of the first fully articulatable arm 168 and second fully articulatable arm 206 with respect to the body 178 by actuation of a single actuator 226.

The outer faces of the pistons 236, 238 may have, for example, concave surfaces to mate with the spherically-shaped portions 172, 204 of the fully articulatable arms 168, 206. The concave surfaces provide for increased contact and superior locking of the fully articulatable arms.

The interior of the housing 174 may include features such as ribs 240 to guide or constrain movement of the pistons 236, 238 along desired translational paths.

It should be appreciated that the housing 174 may restrain movement of the first and second fully articulatable arms 168, 206. For example, the size and shape of the openings 188, 208 in the end caps 180, 200 will to some extent define the range of rotational motion for the respective fully articulatable arms. The edges of the end caps defining the openings could also include a plurality of indentations sized and shaped to receive a portion of the fully articutable arm to define a plurality of preset positions for the fully articulatable arm. It should also be appreciated that the ball joints 176, 217 may, for simplicity, include restraining features in addition to the housing 174.

It should be understood that instead of a single housing and single actuator for the two articulating ball joints 176, 217, the articulating linkage 14 of the present invention could comprise two separate ball joints with separate actuators for locking the joints in desired positions and for unlocking the ball joints to allow full freedom of motion. Use of a single actuator for two articulating ball joints is advantageous in simplifying the locking procedure for the surgeon during placement of the resection guide.

All of the components defining the two ball joints 176, 217 may be made of any commercially available surgical grade material, such as metal, plastic or composite material such as a carbon fiber material. They should preferably be made of a material that can be sterilized by conventional sterilization methods and that will retain their stiffness under the weight of the articulating linkage and guide structure during use. It may be desirable to make the components out of a material that is radio-transparent or radio-lucent so that the components would not block relevant portions of the patient's anatomy intra-operatively when radiographs are taken. To reduce the weight of the instrument set, the components may be made of a lightweight material such as a carbon fiber-polymer composite.

Other embodiments of suitable dual-locking ball joints are disclosed in the following United States patent applications, filed concurrently with United States patent application No. 11/260454, from which this European Patent Application claims priority: Docket No. DEP5368USNP entitled "TRAUMA JOINT, EXTERNAL FIXATOR AND ASSOCIATED METHOD"; Docket No. DEP5558USNP entitled "ORTHOPAEDIC INSTRUMENT JOINT, INSTRUMENT AND ASSOCIATED METHOD"; and Docket No. DEP5559USNP entitled "ORTHOPAEDIC JOINT, DEVICE AND ASSOCIATED METHOD". The complete disclosures of these patent applications are incorporated by reference herein.

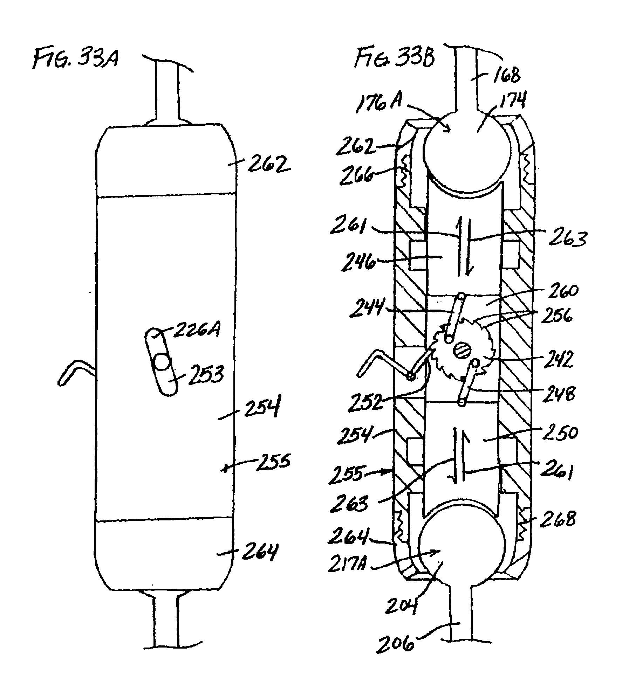

For example, as shown in FIGS. 33A and 33B, a ratchet and lever mechanism could be used in place of the cam structure 234 of FIGS. 10-15 to lock the two ball joints. In FIG. 33, the ball joints are designated 176A and 217A and the actuator mechanism is designated 226A.

In FIGS. 33A and 33B, parts similar to those of the embodiment of FIGS. 10-15 are identified with the same reference numbers; except as otherwise noted, the description of these like parts should be considered applicable to both embodiments. In the embodiment of FIGS. 33A and 33B, the actuator 226A includes a ratchet 242, which is connected by first lever 244 to a first piston 246 and by second lever 248 to second piston 250. A pawl 252 is pivotably connected to body 254 portion of the housing assembly 255. Teeth 256 formed on the ratchet 242 engage the pawl 252.

As the pawl 252 is advanced the actuator 226 is released, permitting the spherical ends 172, 204 of the fully articulatable arms 168, 206 to move freely. Extending from the ratchet 242 is a handle 253 that may be rotated to actuate or lock the ball joints 176A, 217A. By rotating the handle 253 the ball joints 176A, 217A may be locked simultaneously.

The lever arms 244, 248, pistons 246, 250, and the ratchet 242 are received within a cavity 260 of the body 254 of the housing assembly 255. The cavity 260 of the body 254 may, for example, have a generally rectangular or square shape. Such a shape makes possible or eases the use of the actuator 226A that includes the ratchet 242. The pistons 246, 250 can move in the directions of arrows 261, 263 constrained by the surfaces defining the cavity 260. The housing assembly 255 also includes end caps 262, 264 mounted to threaded stubs 266, 268 at each end of the central body 254. The end caps 262, 264 have openings sized and shaped so that the spherical ends 172, 204 of the arms 168, 206 are constrained to be held within the housing but are able to articulate about multiple axes.

The cavity 260 and pistons 246, 250 of the embodiment of FIGS. 33A and 33B have generally rectangular shapes. The pistons 246, 250 are slidably fitted in the cavity 260. The ratchet 242 may be positioned in the cavity 260 and may include a portion, which extends beyond the cavity. For example, the pawl 252 may extend outside the body 254 so that the pawl 252 may be actuated or released and so that the handle 253 may be actuated for locking the ball joints 176A, 217A.

Other variations in the illustrated dual locking ball joint assemblies can be made. For example, instead of the end caps threading onto stubs on the body of the housing assembly, other mechanical locking mechanisms can be used, such as a mating lip and groove.

Next considering the elements of the guide structure 16 of the first illustrated instrument system 10, the guide structure in the first illustrated embodiment comprises a proximal tibial cutting block sized and shaped to be used in resecting the tibial plateau on either the lateral or medial side of the tibia. It should be understood that the present invention is not limited to instruments including proximal tibial cutting blocks; the guide structure 16 could, for example, comprise a distal femoral cutting block. If the instrument is to be used for resecting the bones of other joints, the guide structure could comprise, for example, a proximal femoral cutting guide, a humeral cutting guide or an ankle cutting guide. The guide structure 16 could also comprise a drill or reaming guide for guiding a reamer or drill used to prepare an opening into the intramedullary canal of the bone.

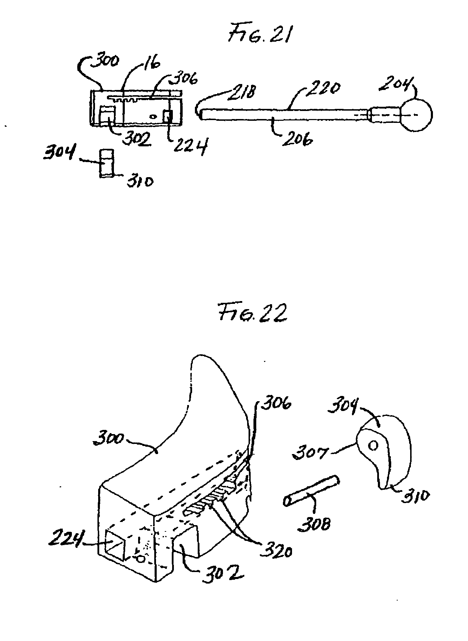

As shown in FIGS. 21-22, the first illustrated guide structure 16 comprises a body 300 with a through-bore 224, a cavity 302 and a guide slot 306; the body 300 is assembled with a cam lock 304 and a pin 308. The illustrated through-bore 224 extends in the anterior-posterior direction through the body 300. The illustrated through-bore is defined by flat surfaces, and is sized and shaped to receive a portion of the shaft 210 of the second fully articulatable arm 206. The cavity 302 is formed in the body 300 adjacent to and in communication with the through-bore 224.

When assembled, the cavity 302 in the body 300 receives the cam portion 307 of the cam lock 304 (see FIGS. 18-19). The cam lock 304 is rotatably mounted to the body 300 through the pin 308. The cam lock 304 also includes a lever 310 exposed outside of the body 300.

As shown in cross-section in FIG. 19, when the cam lock 304 is in an unlocked position, the body 300 of the guide structure 16 can slide along the length of the shaft of the fully articulatable arm 206 in the directions indicated by arrows 312, 314 in FIG. 16. As shown in cross-section in FIG. 18, when the cam lock 304 is rotated to the locked position, the cam 307 bears against the shaft 210 to fix the position of the body 300 on the shaft 210. Thus, the combination of the shapes of the components 210, 300 and the locking mechanism 304 allow for selected translational movement of the guide structure 16 with respect to the second fully articulatable arm 206, but does not allow for rotational movement. The locking mechanism also allows for the translational position of the guide structure 16 to be fixed on the second fully articulatable arm 206.

In the first illustrated embodiment, the guide structure 16 has a slot 306 sized and shaped to receive a cutting blade (such as the serrated blade of the instrument 21 illustrated in FIG. 1A) for resecting the proximal tibia. The illustrated tibial cutting block also includes a plurality of cut-outs 320 to reduce friction between the cutting blade and at least one of the guide surfaces 322 defining the guide slot. It should be understood that a guide slot need not be used; an appropriate tibial cutting block could include a guide surface without any upper constraining surface.

Thus, it can be seen that in the first illustrated instrument set, the guide structure 16 has two translational degrees of freedom of movement and at least eight rotational degrees of freedom of movement with respect to the anchoring structure 12: translational along the anchoring bar 22 and along the shaft 210 of the second fully articulatable arm 206; two rotational degrees of freedom about the movable clamp assembly 28; at least three rotational degrees of freedom about the first ball joint 176; and at least three rotational degrees of freedom about the second ball joint 217. With such freedom on movement available, the surgeon can position a tibial cutting block in the proximal-distal direction to set the resection level, align the cutting block with a desired reference axis and can orient the cutting block in the medial-lateral and anterior-posterior directions to set the varus-valgus orientation and anterior-posterior slope of the resection.

Additional degrees of freedom of movement can be provided in the articulating linkage 14. For example, an additional translational degree of freedom of movement can be provided by using a telescoping member as the length intermediate link 162 instead of the fixed-length bar of the embodiment of FIG. 1. Moreover, an additional rotational degree of freedom of movement can be provided by using a jointed member as the intermediate link 162.

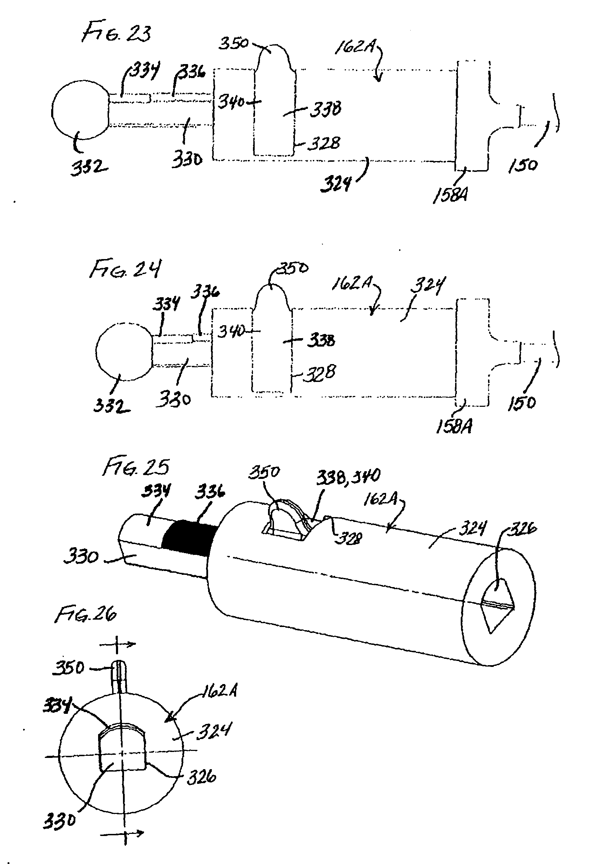

An example of a telescoping member is illustrated in FIGS. 23-32. The telescoping member is illustrated at 162A. It includes a body 324 with a longitudinal through-bore 326 and a slot 328 around part of the periphery of the body 324. As shown in FIG. 25, the illustrated body 324 has a generally cylindrical outer shape, although it should be understood that other shapes may be used. As shown in FIGS. 25-26, the through-bore 326 has three flat sides joined by a curved side along part of its length, although other shapes may be used. At the slot, 328, the through-bore has an expanded volume. The telescoping member 162A also includes a reciprocable rod 330 and a locking mechanism 338. The reciprocable rod 330 has a shape corresponding generally with the shape of the longitudinal through-bore 326, and is capable of being moved in the through-bore 326 along the longitudinal axis of the body 324. As illustrated by FIGS. 23-24, the reciprocable rod 330 can be retracted into or extended out of the body 324. In the illustrated embodiment, the rod 330 corresponds with the first fully articulable arm 168 of the first embodiment: the exposed end of the rod 330 is connected to a spherically-shaped member 332 corresponding with the portion 172 of the first embodiment. The spherically-shaped member 332 can be received in the housing 174 to define the first ball joint when the telescoping member 162A of FIGS. 23-29 is used in place of the intermediate bar 162 of the first embodiment. The opposite end of the body 324 may be connected to a connector 158A, similar to connector 158 of the first embodiment, to connect the telescoping member 162A to the bar portion 150 of the pivotable arm 126.

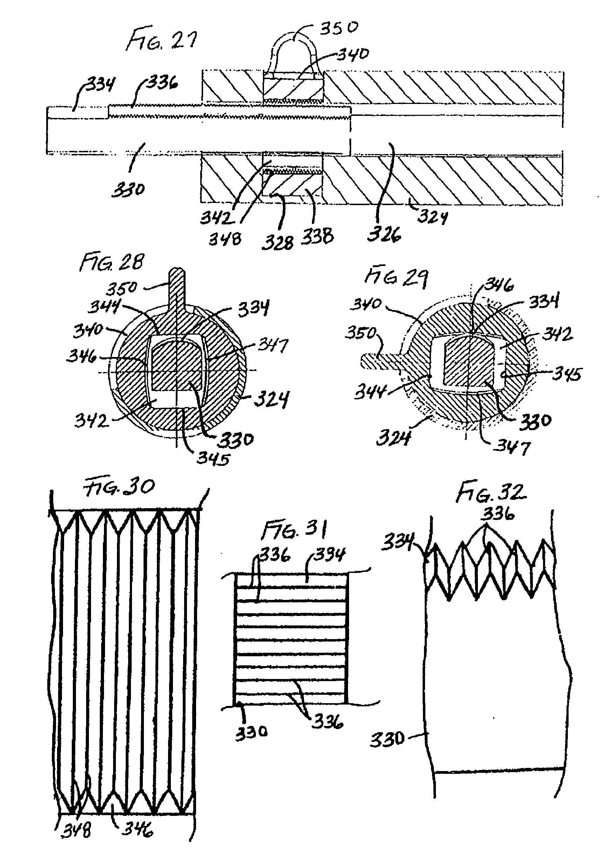

As shown in FIG. 25, the rod 330 in the illustrated telescoping member 162A has a convexly curved upper surface 334 with a plurality of transverse parallel teeth 336 along a substantial part of its length. The remaining four sides of the rod 330 are generally flat, as shown in FIGS. 26 and 29-29.

The illustrated telescoping member 162A also includes a locking mechanism 338. The illustrated locking mechanism 338 has a generally cylindrical ly-shaped outer surface 340, and it mounted in the slot 328 of the body 324. The locking mechanism 338 has a through-bore 342 with two flat parallel sides 344, 345 joined by two curved sides 346, 347. The curved sides 346, 347 have a plurality of parallel teeth 348.

The locking mechanism 338 is rotatable within the body 324 about the longitudinal axis of the housing. The locking mechanism 338 includes a handle 350 extending out of the slot 328 and exposed outside of the body 324. The locking mechanism 338 has an unlocked position shown in FIG. 28 and a locked position shown in FIG. 29. In the unlocked position, the teeth 348 of the locking mechanism 338 are spaced from and do not contact the teeth 336 of the rod 330. In the locked position, the locking mechanism 338 is rotated 90° with respect to the body 324 so that the teeth 348 of the locking mechanism 338 engage the teeth 336 of the rod 330. Thus, in the unlocked position, the rod 330 can be moved to a desired translational position and then locked by rotating the locking mechanism 338 from the position shown in FIG. 28 to that shown in FIG. 29. The locking mechanism 338 can be rotated back to the position shown in FIG. 28 to adjust the length of the telescoping member 162A if desired. It will be appreciated that the number and size of the teeth 336, 348 and distance between the teeth 336, 348 can be selected based upon the desired increments for the overall length of the telescoping member 162A.

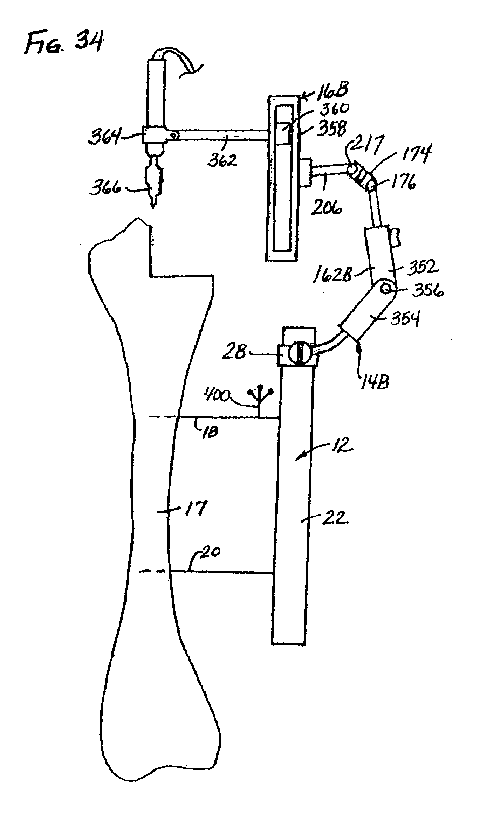

An example of a jointed member that can be used as the intermediate link 162 is illustrated in FIG. 34 at 162B. The intermediate link 162B is an assembly of two segments 352, 354 pivotably joined about a pin or bolt 356. The intermediate link 162B may include any suitable locking mechanism, such as a nut to tighten on the bolt, a locking plate such as that shown in the first embodiment at 134, or a cam locking mechanism or throw so that the two segments 352, 354 can be locked in a particular angular orientation and unlocked and repositioned as desired. Thus, with the intermediate link 162 of FIG. 34, an additional rotational degree of freedom of movement is available in the articulating linkage.

FIG. 34 also illustrates another possible use for the instrument system 10. In FIG. 34, the anchoring structure 12 is again anchored to the femur 17, but the movable clamp assembly 28 is positioned near the proximal end of the anchoring bar 22 and the articulating linkage 14B is oriented toward the proximal end of the femur. As in the first embodiment, the articulating linkage 14B includes two ball joints 176, 217. The guide structure 16B of the embodiment of FIG. 34 is different from that in FIG. 1 in that the guide structure is used to mill, burr or drill into the femur in a proximal-distal direction.

The guide structure 16B of the embodiment of FIG. 33 includes a guide track 358 fixed to the second fully articulatable arm 206. The guide track 358 defines a longitudinal path for a follower 360 and includes an elongate slot through which a support arm 362 extends. One end of the support arm 362 is connected to the follower 360 and the opposite end is connected to a mounting bracket 364. The mounting bracket 364 carries a bone cutting device 366, which in this case may comprise a high speed mill, drill or burr, for example. The surgeon can use the embodiment of FIG. 34 to position the guide track 358 parallel to the axis of the bone 17. The follower 360 can therefore move along the longitudinal path defined by the guide track 358, parallel to the axis of the bone 17, and the support arm, mounting bracket and cutter 366 will move along a parallel linear path. Thus, the instrument system of FIG. 34 also provides freedom of movement along an additional translational path.

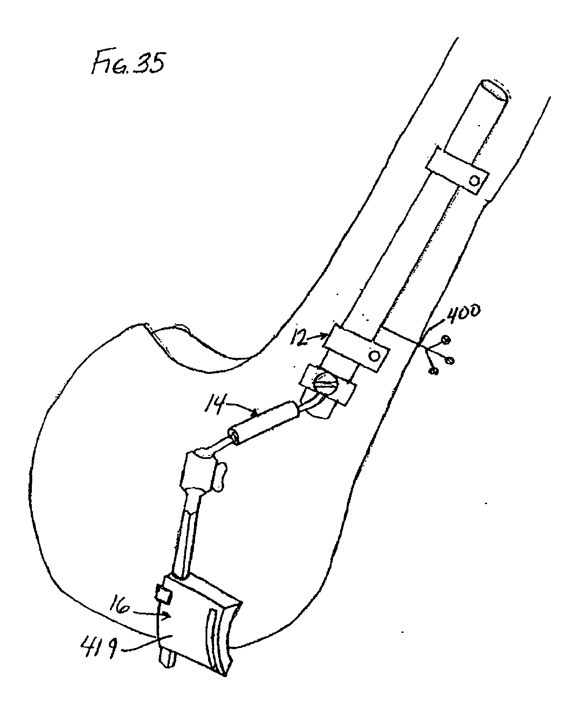

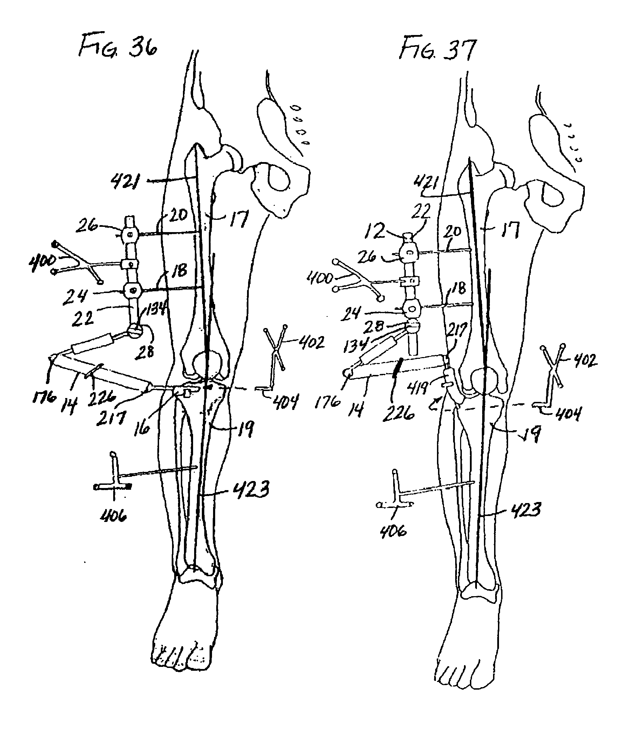

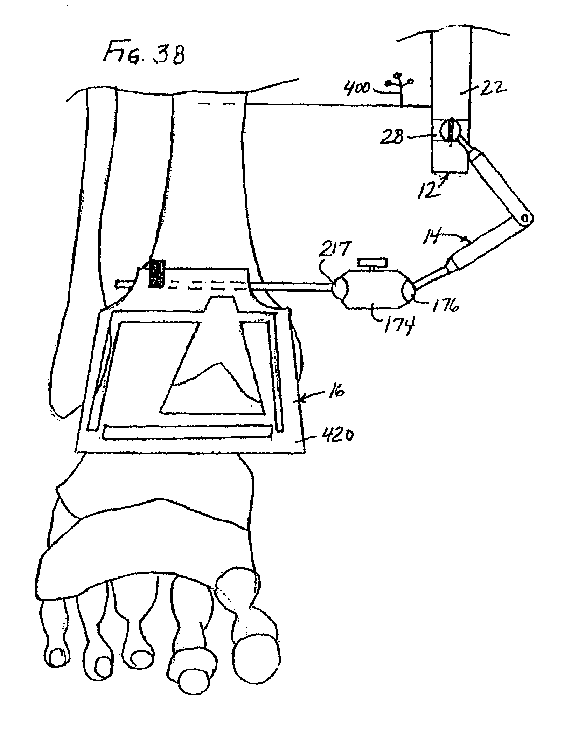

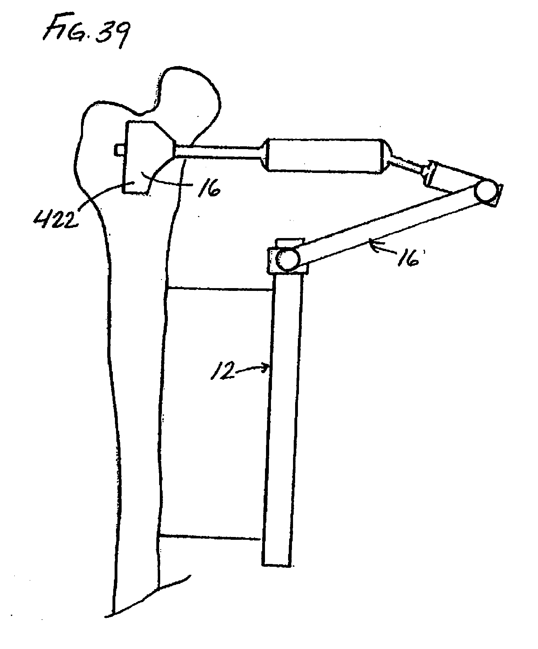

The principles of the present invention could also be applied to other forms of guide structures 16 for resection of other bones. For example, FIGS. 35 and 37 illustrate use of the articulating linkage 14 and anchoring structure 12 of the invention to set the position, alignment and orientation of a distal femoral resection guide shown at 419; in this case, the guide structure 16 comprises the distal femoral cutting guide 419. FIG. 38 illustrates use of the invention in setting the position and orientation of an ankle cutting block 420, thus illustrating another possible guide structure 16 that may be used with the articulating linkage 14. FIG. 39 illustrates use of the invention in setting the position, alignment and orientation of a proximal femoral cutting block 422.

All of the illustrated instrument sets can be used in computer-assisted surgery. As illustrated in FIGS. 34-37, a first computer navigation tracker 400, such as an emitter or reflector array, can be attached to a portion of the anchoring structure 12, such as one of the pins 18, 20 as illustrated in FIG. 34 or the anchoring bar 22 as illustrated in FIGS. 35-37. Suitable fastening mechanisms can be used to secure the arrays 400: for example, a bore could be provided in one of the components of the anchoring structure, such as anchoring bar 22, or clamps, clips or other fastening devices could be employed, as shown in FIGS. 36-37. The instrument system may also include a computer navigation tracker, for example a second emitter or reflector array, such as that shown at 402 in FIGS. 36-37, for mounting to some part of the guide structure 16: for example, the array 402 could be attached to or integral with a plate 404 that is sized and shaped to be received in a cutting guide slot of the guide structure 16. It should be understood that other structures could be employed to attach an array to any of the illustrated guide structures 16. Additional computer navigation trackers, such as those shown at 406 in FIGS. 36-37, could be provided for temporary attachment to one or positions on one of the patient's bones, such as the tibia 19 as shown in FIGS. 36-37. The trackers 400, 402, 406 give the surgeon an image of the position, alignment and orientation of some known part of the guide structure 16, such as a guide slot, with respect to the position, alignment and orientation of other landmarks, such as some part of the anchoring structure 12 or bone that is also displayed on a computer screen. The computer images can be used by the surgeon to guide the guide structure 16 into a desired position, alignment and orientation while the articulating linkage 14 is unlocked, and freely movable; the surgeon can lock the articulating linkage 14 with the guide structure 16 in the desired position, alignment and orientation and then set the guide structure 16 in this position, alignment and orientation by placing standard pins or the like through bores in the guide structure 16 and into the bone. The surgeon may then perform the bone resections so that the bone may receive the prosthetic implant. An example of an emitter or reflector system potentially usable with the present invention is disclosed in U.S. Pat. No. 6,551,325, which is incorporated by reference herein in its entirety. The system 10 of the present invention is expected to be particularly useful with the Ci^{tm} computer assisted surgical system available from DePuy Orthopaedics, Inc. of Warsaw, Indiana. However, any computer assisted surgery system, with appropriate emitters or sensors and computer with appropriate circuitry and programming could be used with the present invention.

The illustrated instrument systems could be used with alternative forms of computer navigation trackers for computer-assisted surgery. For example, instead of an array of emitters or reflectors that is attached to reference points as illustrated in FIGS. 34-37, one or more trackers could be embedded in the guide structure 16 and patient's bone, as well as in the cutting instrument 21. For example, the computer navigation trackers could comprise electromagnetic sensors, such as one or more coils, transducers and transmitters appropriately housed and sealed, and the instrument system could include electromagnetic field generator coils, receiving antenna and computer with appropriate signal receiver and demodulation circuitry. Such systems are commonly referred to as "emat" (electromagnetic acoustic transducer) systems.

Variations from the illustrated embodiments may be made, particularly when the invention is used in with a computer assisted surgical system. For example, the anchoring structure 12 need not be attached to the patient's bone. Instead, the anchoring structure 12 could comprise a fixture in the operating room, such as a rod or bar fixed to the operating table or a dedicated floor stand.

Although the present invention provides advantages in computer-assisted surgery, its use is not limited to computer-assisted surgery. The guide structure 16, articulating structure 14 and anchoring structure 12 could also be used with standard surgical instruments used to determine position, alignment and orientation, such as a stylus or an extramedullary or intramedullary alignment rod. FIGS. 36 and 37 illustrate extramedullary alignment rods at 421 and 423.

It will be appreciated from a comparison of FIGS. 36 and 37 that the system of the present invention is advantageous in that the same anchoring structure 12 and articulating linkage 14 can be used to position, alignment and orient cutting blocks for use in all bones of a joint; the surgeon need only change the guide structure 16. In FIG. 36, the guide structure 16 is a proximal tibial cutting block and in FIG. 37 the guide structure 16 is a distal femoral cutting block. Moreover, as can be seen in FIGS. 36 and 37, the surgeon need not change the position of the anchoring structure 12 when using the system for setting multiple cutting blocks. Accordingly, a kit incorporating the system of the present invention may include a single articulating linkage 14 and multiple guide structures 16 for resecting multiple bones of a joint.

A method of using the illustrated surgical instrument system 10 in surgery is described below.

The patient is placed supine on the operating table and given a satisfactory anesthetic. The leg or other limb is prepped and draped in the usual fashion. The anchoring structure 12 is then set in position. As in FIGS. 1 and 36-37, the anchoring structure 12 can be set by driving the pins 18, 20 into a bone (such as the femur) and fixing the anchoring clamp assemblies 24, 26 to the pins 18, 20. The anchoring bar 22, with the movable clamp assembly 28 and remainder of the articulating linkage 14 mounted on the bar 22, can then be fixed to the anchoring clamp assemblies 24, 26. At this point, the movable clamp assembly 28 need not be locked in any translational position and the remaining locks 134, 226 of the articulating assembly may be in the unlocked state.

It will be appreciated that in some environments, the stationary structure need not be attached to the patient. For example, the stationary structure could comprise an operating room fixture, in which case the surgeon would attach the movable clamp assembly 28 to the operating room fixture.

The surgeon selects the appropriate guide structure 16 and slides the fully articulatable arm 206 into the cavity 302 of the guide structure 16. It will be appreciated that a kit including the system 10 may include two or more sizes of each type of guide structure 16 to accommodate the needs of different patients.

If the procedure includes the use of a computer to position, align and orient the resection guide 16, computer navigation tracker 402 can be mounted to the resection guide 16 by sliding the plate 404 into the cutting guide slot (such as slot 306 shown in FIG. 16) of the guide structure 16; alternatively, a computer navigation tracker could be embedded within the guide structure 16. Other computer navigation trackers 400, 406 may be affixed to anatomical landmarks (or embedded within the patient's bone) or to the anchoring structure 12 to serve as references or benchmarks for determining the relative position, alignment and orientation of the guide structure 16.

The surgeon can then move the guide structure 16 into a desired position, alignment and orientation and begin locking the lock mechanisms. For example, once the surgeon is satisfied with the general location of the guide structure 16, the translational position of the movable clamp assembly 28 on the bar 22 can be fixed by tightening the bolt 114. The surgeon can then continue to lock the articulating linkage 14; for example, the surgeon may next turn the handle 136 of the locking plate 134 to fix the position of the pivotable arm 126 with respect to the anchoring structure 12. The translational position of the guide structure 16 on the shaft of the second fully articulatable arm 206 may be fixed by pushing on the lever 310 of cam lock 304. At this point, orientation of the guide structure may still be adjusted since the two ball joints 176, 217 can still articulate in three rotational degrees of freedom. The surgeon can set the orientation (varus-valgus angle and the anterior-posterior slope in a knee arthroplasty such as illustrated in FIGS. 36 and 37) and then lock the resection guide 16 in place by turning the actuator 226 so that the pistons 236, 238 simultaneously engage the spherical portions 172, 204 of the ball joints 176, 217, thus completing the locking process. When the locking process is complete, the articulating linkage 14 is substantially rigid and the guide structure 16 is fixed in position with respect to the anchoring structure 12. If the surgeon is satisfied with the final fixed position, alignment and orientation and of the guide structure 16, the surgeon can place pins through receiving holes in the guide structure and into the underlying bone (if the guide structure comprises a resection guide). Throughout this process, the surgeon can monitor the position, alignment and orientation of the guide structure on a monitoring device such as a computer screen. The computer navigation tracker 402 can be removed from the guide structure 16 once the surgeon is satisfied with its location.

It will be appreciated that if at any time the surgeon is dissatisfied with the location of the guide structure 16, one or more of the locking mechanisms 134, 226, 304 can be unlocked for repositioning of the guide structure 16 followed by locking.

In the case of resection guides, it will also be appreciated that if the articulating linkage 14 is obstructing the surgeon in any way, once the resection guide is fixed to the bone with pins, the cam lock 304 can be unlocked and the second fully articulatable arm 206 can be pulled out of the bore 224 and the articulating linkage 14 can be moved out of the way by unlocking one or more of the locks. However, it should not be necessary to remove the articulating linkage 14 from the anchoring structure 12.

The surgeon can then perform bone resections using a cutting instrument such as shown at 19 in FIG. 1A, for example. Other cutting instruments, such as a rotating burr could also be used. Once the resections of this first bone are complete, the surgeon can select another guide structure 16 (such as a distal femoral resection guide 419 as shown in FIG. 37) designed for resection of the other bone of the joint and mount this guide structure 16 on the second fully articulatable arm 206. With the locking mechanisms 134, 226, 304 disengaged, the surgeon can then move the second guide structure 16 into a desired position, alignment and orientation with respect to the second bone of the joint, lock the articulating linkage once the position, alignment and orientation are set, fix the second guide structure to the second bone and make the desired resections. Setting of the position, alignment and orientation of the second guide structure can be computer guided as well. All of the steps involving the first and second resection guides can be performed with a single articulating linkage 14 fixed to a single anchoring structure 12, and can be performed without moving the anchoring structure 12 and without totally disengaging the articulating linkage 14 from the anchoring structure 12.

It will be appreciated that if the articulating linkage 14 includes features such as the telescoping member 162A, the surgeon would also adjust and lock the telescoping member at some time during placement of the guide structure 16. Similarly, if a jointed member 162B such as that shown in FIGS. 34 and 38 is used as part of the articulating linkage, then the surgeon would also adjust and lock the jointed member 162B at some time during placement of the guide structure. If the two ball joints 176, 217 do not use a dual-locking mechanism such as that illustrated in FIGS. 11, 13-14 or 33, the step of locking the ball joints 176, 217 would be a two-step process. It will also be appreciated that although the surgical technique described above relates particularly to knee arthroplasty, the same general steps can be followed for performing other resections at other joints. It will also be appreciated that instead of using computer navigation trackers, the surgical technique could employ standard mechanical alignment devices (such as alignment rods) and standard anchoring structures such as ankle clamps.

## Claims

**1.** A surgical instrument system for resecting a bone during joint arthroplasty, the system comprising:
a cutting instrument;
an anchoring structure;
a guide structure for guiding the path of the cutting instrument; and
an articulating linkage connecting the anchoring structure to the guide structure, the articulating linkage including a plurality of lockable ball joints arranged in series, each ball joint providing for freedom of movement about three axes of rotation.

**2.** The surgical instrument system of claim 1 wherein the anchoring structure includes a set of pins, a bar and a pair of mounting clamps for fixing the bar to the pins.

**3.** The surgical instrument system of claim 2 wherein the articulating linkage includes a movable clamp mountable on the bar and an arm mounted to the movable clamp for rotational movement about two axes, the movable clamp being capable of being moved linearly along the bar and rotationally about the bar to a desired position and fixed in the desired position, the arm being capable of being fixed in a desired rotational position with respect to the movable clamp.

**4.** The surgical instrument system of claim 3 wherein the arm has two degrees of rotational freedom with respect to the movable clamp.

**5.** The surgical instrument system of claim 1 wherein the articulating linkage includes a telescoping member.

**7.** The surgical instrument system of claim 1 wherein the guide structure includes a flat cutting guide surface and a plurality of spaced apertures for receiving pins.

**8.** The surgical instrument system of claim 7 wherein the guide structure includes a cutting guide slot.

**9.** The surgical instrument system of claim 1 wherein the guide structure includes a slot and a movable cam.

**10.** The surgical instrument system of claim 9 wherein the articulating linkage includes a rod received in the slot, and wherein the movable cam is movable to a position wherein the cam contacts the rod of the articulating linkage.

**11.** The surgical instrument system of claim 10 wherein the guide structure has a degree of translational freedom of movement along the rod of the articulating linkage.

**12.** The surgical instrument system of claim 1 further comprising a plurality of computer navigation trackers.

**13.** The surgical instrument system of claim 12 wherein the guide structure comprises a cutting block having a cutting guide slot and at least one of the computer navigation trackers includes a plate sized and shaped to be received in the cutting guide slot of the guide structure.

**14.** The surgical instrument system of claim 12 wherein at least one of the computer navigation trackers comprises an electromagnetic sensor.

**15.** The surgical instrument system of claim 1 further comprising a second guide structure for guiding the path of the cutting instrument, and wherein the first guide structure is detachable from the articulating linkage and the second guide structure is attachable to the articulating linkage.

**16.** The surgical instrument system of claim 15 wherein the first guide structure comprises a tibial cutting block and the second guide structure comprises a femoral cutting block.

**17.** The surgical instrument system of claim 1 wherein the cutting instrument is selected from the group consisting of a saw, a milling device, a burr and a drill.
